# EUROPÄISCHE PATENTSCHRIFT

(11) **EP 1 594 455 B1**
(45) Veröffentlichungstag und Bekanntmachung des Hinweises auf die Patenterteilung: **11.06.2014**
(21) Anmeldenummer: 04701960.9
(22) Anmeldetag: 14.01.2004
(51) Int. Cl.: A61K 8/44, A61K 8/49, A61Q 7/00, A61Q 17/04, A61Q 19/08

(54) **KOSMETISCHE ODER DERMATOLOGISCHE ZUBEREITUNGEN MIT EINEM GEHALT AN KREATIN, KREATININ UND/ODER SEINEN DERIVATEN IN KOMBINATION MIT SOJABOHNENKEIMEXTRAKTEN**
COSMETIC OR DERMATOLOGICAL PREPARATIONS CONTAINING CREATINE, CREATININE AND/OR THE DERIVATIVES THEREOF COMBINED WITH SOYA BEAN EXTRACTS
PREPARATIONS COSMETIQUES OU DERMATOLOGIQUES CONTENANT DE LA CREATINE, DE LA CREATININE ET / OU LEURS DERIVES EN COMBINAISON AVEC DES EXTRAITS DE GERME DE GRAINES DE SOJA

(30) Priorität: 17.01.2003 DE 10301632
(43) Veröffentlichungstag der Anmeldung: 16.11.2005
(73) Patentinhaber: Beiersdorf AG, 20253 Hamburg (DE)
(72) Erfinder: STAEB, Franz, 21379 Echem (DE); BLATT, Thomas, 22880 Wedel (DE); SCHMIDT, Melanie, 21031 Hamburg (DE); MUNDT, Claudia, 28207 Bremen (DE); GALLINAT, Stefan, 22880 Wedel (DE); VENZKE, Kirsten, 26725 Emden (DE); LENZ, Holger, 22529 Hamburg (DE); MEIER-ZIMMERER, Cornelia, 22529 Hamburg (DE)
(86) Internationale Anmeldenummer: PCT/EP2004/050015
(87) Internationale Veröffentlichungsnummer: WO 2004/064801

(56) Entgegenhaltungen:
- WO-A-98/53704
- WO-A-03/011241
- DE-A- 19 841 385
- US-B1- 6 455 032
- DATABASE WPI Section Ch, Week 200031 Derwent Publications Ltd., London, GB; Class B04, AN 2000-353617 XP002282453 & JP 2000 109420 A (POLA CHEM IND INC), 18. April 2000 (2000-04-18)

## Beschreibung

Die vorliegende Erfindung betrifft die Verwendung von Kombinationen von Kreatinin mit Kreatin mit Sojabohnenkeimextrakten in kosmetischen oder dermatologischen Zubereitungen zur Behandlung und Prophylaxe der Symptome von UV- und oder Ozon- induzierten Hautschäden sowie von entzündlichen und degenerativen Hautzuständen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüs-sen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).

Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomene.

Produkte zur Pflege gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Daüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Insbesondere betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)), nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzliche Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der Erfindung war es daher auch, kosmetische, dermatologische und pharmazeutische Wirkstoffe und Zubereitungen sowie Lichtschutzformulierungen zu schaffen, die zur Prophylaxe und Behandlung lichtempfindlicher Haut, insbesondere Photodermatosen, bevorzugt PLD dienen.

Weitere Bezeichnungen für die polymorphe Lichtdermatose sind PLD, PLE, Mallorca-Akne und eine Vielzahl von weiteren Bezeichnungen, wie sie in der Literatur (z.B. A. Voelckel et al, Zentralblatt Haut- und Geschlechtskrankheiten (1989), 156, S.2), angegeben sind.

Hauptsächlich werden Antioxidantien als Schutzsubstanzen gegen den Verderb der sie enthaltenden Zubereitungen verwendet. Dennoch ist bekannt, daß auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/Californien), werden oxidative Schäden der Haut und ihre näheren Ursachen aufgeführt.

Auch aus dem Grunde, solchen Reaktionen vorzubeugen, können kosmetischen oder dermatologischen Formulierungen zusätzlich Antioxidantien und/oder Radikalfänger einverleibt werden.

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der vorliegenden Erfindung war es somit, Wege zu finden, die die Nachteile des Standes der Technik vermeiden. Insbesondere soll die Wirkung der Behebung der mit der endogenen, chronologischen und exogenen Hautalterung verbundenen Schäden und die Prophylaxe dauerhaft, nachhaltig und ohne das Risiko von Nebenwirkungen sein.

US 6 455 032 B1 offenbart Zubereitungen zur Behandlung von lichtbeschädigter Haut enthaltend Isoflavone oder Sojaextrakte.

Es war indes überraschend und für den Fachmann nicht vorauszusehen, daß kosmetische oder dermatologische Zubereitungen mit einem Gehalt an einer Wirkstoffkombination aus Kreatinin mit Kreatin und Sojabohnenkeimextrakten den Nachteilen des Standes der Technik abhelfen.

Bei Anwendung der erfindungsgemäß verwendeten Wirkstoffkombination bzw. kosmetischer oder topischer dermatologischer Zubereitungen mit einem wirksamen Gehalt an erfindungsgemäßer Wirkstoffkombination ist in überraschender Weise eine wirksame Behandlung, aber auch eine Prophylaxe
- von defizitären, sensitiven oder hypoaktiven Hautzuständen oder defizitären, sensitiven oder hypoaktiven Zustände von Hautanhangsgebilden
- von Erscheinungen vorzeitiger Alterung der Haut (z.B. Falten, Altersflecken, Teleangiektasien) und/oder der Hautanhangsgebilde,
- von umweltbedingten (Rauchen, Smog, reaktive Sauerstoffspecies, freie Radikale) und insbesondere lichtbedingten negativen Veränderungen der Haut und der Hautanhangsgebilde.
- von lichtbedingten Hautschäden
- von Pigmentierungsstörungen,
- von Juckreiz,
- von trockenen Hautzuständen und Hornschichtbarrierestörungen,
- von Haarausfall und für verbessertes Haarwachstum
- von entzündlichen Hautzuständen sowie atopischem Ekzem, seborrhoischem Ekzem, polymorpher Lichtdermatose, Psoriasis, Vitiligo
   möglich. Die erfindungsgemäße Wirkstoffkombination bzw. kosmetische oder topische dermatologische Zubereitungen mit einem wirksamen Gehalt an erfindungsgemäßer Wirkstoffkombination dient aber auch in überraschender Weise
- zur Beruhigung von empfindlicher oder gereizter Haut
- zur Stimulation der Kollagen-, Hyaluronsäure-, Elastinsynthese
- zur Stimulation der intrazellulären DNA-Synthese, insbesondere bei defizitären oder hypoaktiven Hautzuständen.
- zur Steigerung der Zellerneuerung und Regeneration der Haut
- zur Steigerung der hauteigenen Schutz- und Reparaturmechanismen (beispielsweise für dysfunktionelle Enzyme, DNA, Lipide, Proteine)
- zur Vor- und Nachbehandlung bei topischer Anwendung von Laser- und Abschleifbehandlungen, die z. B. der Reduzierung von Hautfalten und Narben dienen, um den resultierenden Hautreizungen entgegenzuwirken und die Regenerationsprozesse in der verletzten Haut zu fördern.

Bevorzugt ist es, wenn der Gehalt an Kreatinin 0,001 - 50 Gew.-% , vorzugsweise 0,1 -10 Gew.-% sowie der Gehalt an Kreatin 0,001 - 50 Gew.-% , vorzugsweise 0,1 - 10 Gew.-%, bezogen auf das Gesamtgewicht der Zubereitungen, beträgt.

Bevorzugt ist es, wenn die Zubereitungen 0,1 bis 20 Gew.-%, vorteilhaft 0,5 bis 10 Gew.-%, ganz besonders bevorzugt 1 bis 5 Gew.-% Sojabohnenkeimextrakte enthalten.

Erfindungsgemäße Zubereitungen werden bevorzugt verwendet zur Restrukturierung und/oder Verjüngung der Haut, zur Erleichterung der Vernarbung bei verringerter Narbenbildung bei Verletzungen der Oberhaut sowie zur Inspiration des Körpers und der Sinne des Menschen.

Die Erfindung umfasst auch die Verwendung solcher Zubereitungen zur Prophylaxe und/oder Behandlung von entzündlichen Hautzuständen und/oder zum Hautschutz bei empfindlich determinierter und trockener Haut (wie z. B. atopisches Ekzem, seborrhoisches Ekzem, polymorphe Lichtdermatose, Psoriasis, Vitiligo, Wundheilungsstörungen, Juckreiz, empfindlicher oder gereizter Haut, lichtbedingte Hautschäden und UV-induzierte Immunsuppression, Veränderungen der Desquamation, Veränderungen der normalen Fibroblasten-und Keratinozytenproliferation,Veränderungen der normalen Fibroblasten- und Keratinozytendifferenzierung defizitären sensitiven oder hypoaktiven Hautzustände oder defizitären sensitiven oder hypoaktiven Zustände von Hautanhangsgebilden und zur Verringerung der Hautdicke).

Die Erfindung umfasst auch die Verwendung solcher Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut. (wie z. B. degenerative Erscheinungen der Haut (wie Altersflecken, Falten, Teleangiektasien, Hautschlaffheit, Elastizitätsverlust, sowie Schwund der epidermalen und dermalen Zellschichten, der Bestandteile des Bindegewebes, der Retezapfen und Kapillargefässe), sogenannte Skin luster und fatigue (Hauterschlaffung und/oder Hautermüdung), gesteigerte Aktivierung proteolytischer Enzyme in der Haut wie z. B. Metalloproteinasen, Störungen der normalen Kollagen-, Hyaluronsäure-, Elastin- und Glykosaminoglycan-Homeostase und der normalen Hautregeneration, Veränderungen der normalen Fibroblasten- und Keratinozytenproliferation,Veränderungen der normalen Fibroblasten- und Keratinozytendifferenzierung, Mangelerscheinungen der intrazellulären DNS-Synthese (insbesondere bei defizitären oder hypoaktiven Hautzuständen), umweltbedingte (durch Rauchen, Smog, reaktive Sauerstoffspecies, freie Radikale und dergleichen verursachte) negative Veränderungen der Haut und/oder der Hautanhangsgebilde).

Die Erfindung umfasst auch die Verwendung solcher Zubereitungen zur Behandlung und/oder Prophylaxe von Pigmentierungsstörungen, zur Steigerung der Ceramidbiosynthese (wie z.B. Veränderungen des Ceramid-, Lipid- und Energiestoffwechsels der gesunden Haut), zur Stärkung der Barrierefunktion der Haut (wie z. B. Hornschichtbarrierestörungen, Veränderungen der normalen Lipidperoxidationen, Veränderungen des transepidermalen Wasserverlustes und des normalen Feuchtigkeitsgehaltes der Haut), zur Behandlung und/oder Prophylaxe von Störungen des normalen Haut-pH-Werts und der Osmolytbalance, zur Behandlung und/oder Prophylaxe von Abweichungen von der normalen Zell-Zell-Kommunikation in der Haut (z.B. interzelluläre Kommunikationen über Mediatoren und/ oder über mechanische/ physiologische Verbindungen).

Die Erfindung umfasst auch die Verwendung solcher Zubereitungen zur Behandlung und/oder Prophylaxe von funktionellen Störungen der Hautanhangsgebilde (z.B. Haarausfall, verbessertes Haarwachstum, seborrhoischer Erscheinungen, fettige Haut, fettiges Haar, Komedonen, aber auch Kopfschuppen).

Erfindungsgemäße Effekte liegen in der Stabilisierung des Energiestoffwechsels im Sinne eines Synergismus zwische den erfindungsgemäßen Komponenten Kreatin, Kreatinin und Sojabohnenkeimextrakten im Vergleich zu Kreatin und Kreatinin allein. Durch die verbesserte Energielage der Hautzellen kann eine wesentliche bessere Wirkung zur Prophylaxe und Behandlung von degenerativen Haut- und Haarerscheinungen (Falten, Erschlaffung,

Degeneration, Altersflecken, Durchblutungsstörungen, Juckreiz, Stressempfindlichkeit, Sprödigkeit) erzielt werden.

Kreatinin (von grch.: τo κρεα 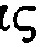 = "das Fleisch") ist durch folgende Struktur gekennzeichnet 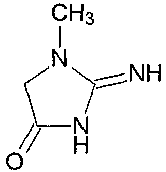 und entsteht im Organismus durch nichtenzymatische Umwandlung aus Kreatinphosphat gemäß 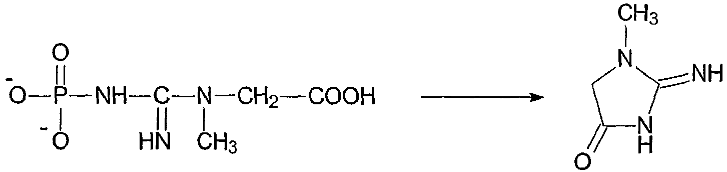 und wird über die Niere ausgeschieden. Die Menge der Kreatininausscheidung ist der Muskelmasse proportional und für das jeweilige Individuum annähernd konstant. Kreatinin ist in Fleischextrakt und Fleischbrühwürfeln enthalten.

Kreatin (ebenfalls von grch.: τo κρεα 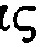 = "das Fleisch") zeichnet sich durch folgende Struktur aus:

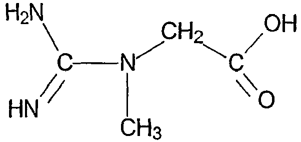

Es findet sich im Muskelsaft der Wirbeltiere zu 0,05-0,4%, in geringen Mengen auch im Gehirn und Blut. Als Monohydrat stellt es ein farbloses, kristallines Pulver dar. In wäßriger Lösung wird Kreatinin gebildet. Im Organismus entsteht es durch Transamidinierung von L-Arginin auf Glycin zu Guanidinoessigsäure und deren anschließende Methylierung mittels S-Adenosylmethionin (durch Guanidinoacetat-Methyltransferase). Man hält Kreatin für einen appetitfördernden Bestandteil von Rindfleisch und Fleischextrakt. Kreatinzusatz zur Nahrung verstärkt die körperliche Leistungsfähigkeit.

Bevorzugt ist es von Vorteil, das Gewichtsverhältnis von Kreatinin zu Kreatin aus dem Bereich von 50 : 1 bis 1 : 50, bevorzugt von 10 : 1 bis 1 : 10, insbesondere bevorzugt von 2 : 1 bis 1 : 2 zu wählen.

Kreatinphosphat, weist folgende Struktur auf. 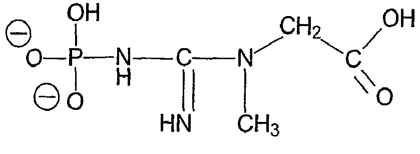 und ist im frischen Muskel verbreitet ist und spielt dort als energiespeicherndes Phosphat (Phosphagen) eine wichtige Rolle Im arbeitenden Muskel gibt Kreatinphosphat mit Adenosin-5'-diphosphat unter dem Einfluß des Enzyms Kreatin-Kinase Adenosin-5'-triphosphat (ATP) und Kreatin; im ruhenden Muskel läuft die umgekehrte Reaktion ab.
Aber auch Kreatinsulfat, Kreatinacetat, Kreatinascorbat und die an der Carboxylgruppe mit mono- oder polyfunktionalen Alkoholen veresterten Derivate führen zu vorteilhaften Ausführungsformen der Erfindung.

Sojabohnenkeimextrakte, die unter anderem Daidzin, Glycitin, Genistin, Daidzein, Glycitein, Genistein sowie Saponine enthalten sind beispielsweise kommerziel erhältlich bei der Gesellschaft L.M Cosmetics, Thiais, Frankreich unter der Bezeichnung Isoflavones 150 (im folgenden auch als Isoflavon 150 bezeichnet). Diese Mischung enthält beispielsweise 39,01 ppm Daidzin, 1,53 ppm Malonyldaidzin, 20,56 ppm Acetyldaidzin, 22,47 ppm Glycitin, 1,74 ppm Malonylglycitin, 12,69 ppm Acetylglycitin, Genistin, 1,43 ppm Daidzein, 9,82 ppm Glycitein, 9,82 ppm Genistin, 0,1 ppm Malonylgenistin, 5,7 ppm Acetylgenistin, 0,33 ppm Genistein.

Es ist erfindungsgemäß insbesondere äußerst vorteilhaft, die erfindungsgemäß verwendete Wirkstoffkombination bzw. kosmetische oder topische dermatologische Zubereitungen mit einem wirksamen Gehalt an erfindungsgemäß verwendeter Wirkstoffkombination zur kosmetischen oder dermatologischen Behandlung oder Prophylaxe unerwünschter Hautzustände zu verwenden.

Erfindungsgemäß können Zubereitungen, welche die erfindungsgemäßen Wirkstoffkombinationen enthalten, übliche Antioxidantien eingesetzt werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnösin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Vitamin C und Derivate (z.B. As-corbylpalmitat, Mg-Ascorbylphosphat, Ascorbylacetat), Tocopherole und Derivate (z.B. Vitamin-E-acetat), sowie Koniferylbenzoat des Benzoëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Prophylaxe bzw. die kosmetische oder dermatologische Behandlung mit dem erfindungsgemäß verwendeten Wirkstoffkombinationen bzw. mit den kosmetischen oder topischen dermatologischen Zubereitungen mit einem wirksamen Gehalt an erfindungsgemäß verwendeter Wirkstoffkombination erfolgt in der üblichen Weise, und zwar dergestalt, daß der erfindungsgemäß verwendete Wirkstoff bzw. die kosmetischen oder topischen dermatologischen Zubereitungen mit einem wirksamen Gehalt an erfindungsgemäß verwendetem Wirkstoff auf die betroffenen Hautstellen aufgetragen wird.

Vorteilhaft kann die erfindungsgemäß verwendete Wirkstoffkombination eingearbeitet werden in übliche kosmetische und dermatologische Zubereitungen, welche in verschiedenen Formen vorliegen können. So können sie z.B. eine Lösung, eine Emulsion vom Typ Wasserin-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O), eine Hydrodispersion oder Lipodispersion, ein Gel, einen festen Stift oder auch ein Aerosol darstellen.

Erfindungsgemäße Emulsionen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, die erfindungsgemäß verwendete Wirkstoffkombination in wäßrige Systeme bzw. Tensidzubereitungen zur Reinigung der Haut und der Haare einzufügen.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, die erfindungsgemäß verwendete Wirkstoffkombination als Zusatzstoff zu Zubereitungen zu geben, die bereits andere Wirkstoffe für andere Zwecke enthalten.

Entsprechend können kosmetische oder topische dermatologische Zusammensetzungen im Sinne der vorliegenden Erfindung, je nach ihrem Aufbau, beispielsweise verwendet werden als Hautschutzcreme, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Günstig sind gegebenenfalls auch solche kosmetischen und dermatologischen Zubereitungen, die in der Form eines Sonnenschutzmittels vorliegen. Vorzugsweise enthalten diese neben der erfindungsgemäß verwendeten Wirkstoffkombination zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoesäure-Derivate, vorzugsweise 4-(Dimethylamino)-benzoesäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxyben-zophenon-5-sulfonsäure und ihre Salze;
- Sulfonsäure-Derivate des 3-Benzylidencamphers, wie z.B. 4-(2-Oxo-3-bornyliden-methyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäßen kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl-oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropyl-myristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Die nachfolgenden Beispiele sollen die vorliegende Erfindung verdeutlichen.

### 1. PIT Emulsionen

Im Labor Maßstab werden die Fett- und Wasserphase getrennt auf 80°C erwärmt. Die Fettphase wird vorgelegt. Bei 80°C wird das Parfüm zugegeben, anschließend wird die Wasserphase zugefügt. Die Emulsion wird unter rührend auf Raumtemperatur abgekühlt. Eine Homogenisierung ist aufgrund der spontanen Bildung der Emulsion nicht erforderlich.

| **Beispiele** | **1** | **2** | **3** | **4** | **5** |
|---|---|---|---|---|---|
| Glycerinmonostearat selbstemulqierend | 0,50 | | 3,00 | 2,00 | 4,00 |
| Polyoxyethylen(12)cetylstearylether | | 5,00 | | 1,00 | 1,50 |
| Polyoxyethylen(20)cetylstearylether | | | | 2,00 | |
| Polyoxyethylen(30)cetylstearylether | 5,00 | | 1,00 | | |
| Stearylalkohol | | | 3,00 | | 0,50 |
| Cetylalkohol | 2,50 | 1,00 | | 1,50 | |
| 2-Ethylhexyl Methoxyzinnamat | | | | 5,00 | 8,00 |
| 2,4-Bis-(4-(2-ethyl-hexyloxy-)2-hydroxyl)-phenyl)-6-(4-methoxyphenyl)-(1,3,5)-triazin | | 1,50 | | 2,00 | 2,50 |
| 1-(4-tert-Butylphenyl)-3-(4-methoxyphenyl)-1,3-propandion | | | 2,00 | | |
| Diethylhexyl Butamidotriazon | 1,00 | 2,00 | | 2,00 | |
| Ethylhexyl Triazon | 4,00 | | 3,00 | 4,00 | |
| 4-Methylbenzyliden Campher | | 4,00 | | | 2,00 |
| Octocrylen | | 4,00 | | | 2,50 |
| Phenylen-1,4-bis-(mononatrium, 2-benzimidazyl-5,7-disulfonsaeure | | | 0,50 | | 1,50 |
| Phenylbenzimidazol Sulfonsäure | 0,50 | | | 3,00 | |
| C12-15 Alkyl Benzoat | | 2,50 | | | 5,00 |
| Titandioxid | 0,50 | 1,00 | | 3,00 | 2,00 |
| Zinkoxid | 2,00 | 3,00 | 3,00 | 0,50 | 1,00 |
| Dicaprylylether | | | 3,50 | | |
| Butylenglycol-Dicaprylat/-Dicaprat | 5,00 | | | 6,00 | |
| Dicaprylylcarbonat | | | 6,00 | | 2,00 |
| Dimethicon Polydimethylsiloxan | | 0,50 | 1,00 | | |
| Phenylmethylpolysiloxan | 2,00 | | | 0,50 | 0,50 |
| Shea-Butter (Sheabutter) | | 2,00 | | | 0,50 |
| PVP Hexadecencopolymer | 0,50 | | | 0,50 | 1,00 |
| Glycerin | 3,00 | 7,50 | 5,00 | 7,50 | 2,50 |
| Tocopherolacetat | 0,50 | | 0,25 | | 1,00 |
| Isoflavon-150 | 0,10 | 0,20 | 0,20 | 0,50 | 0,10 |
| Kreatinin | 0,30 | 0,10 | 0,60 | 0,20 | 0,30 |
| Kreatin | 0,30 | 0,10 | 0,60 | 0,20 | 0,30 |
| Alpha-Glucosylrutin | 0,10 | | 0,20 | | |
| Konservierungsmittel | q.s. | q.s. | q.s. | q.s. | q.s. |
| Ethanol | 3,00 | 2,00 | 1,50 | | 1,00 |
| Parfum | q.s. | q.s. | q.s. | q.s. | q.s. |
| Wasser | ad. 100 | ad. 100 | ad. 100 | ad. 100 | ad. 100 |

### 2. O/W-Creme

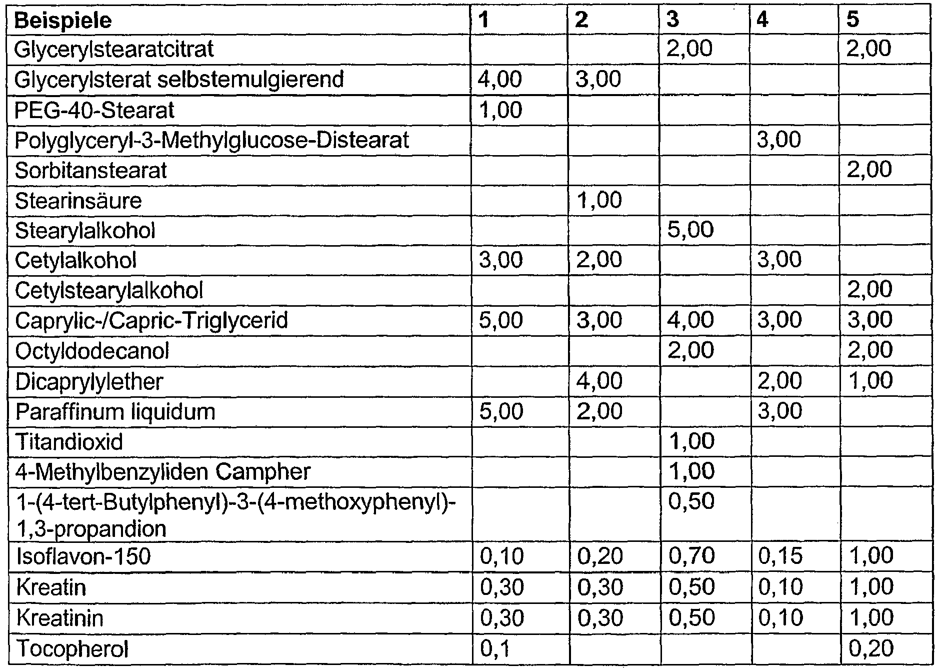

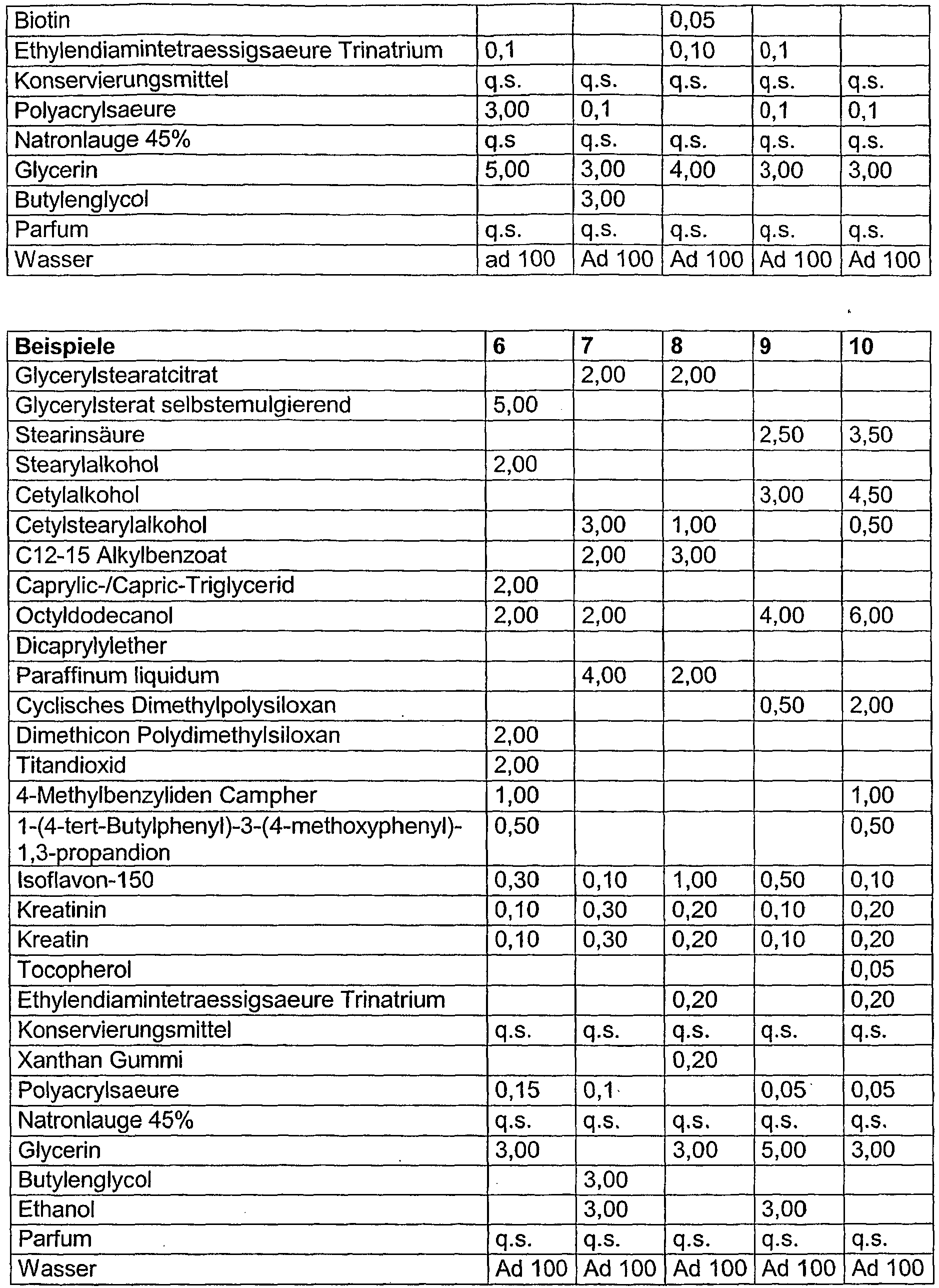

### 3. W/O-Emulsionen

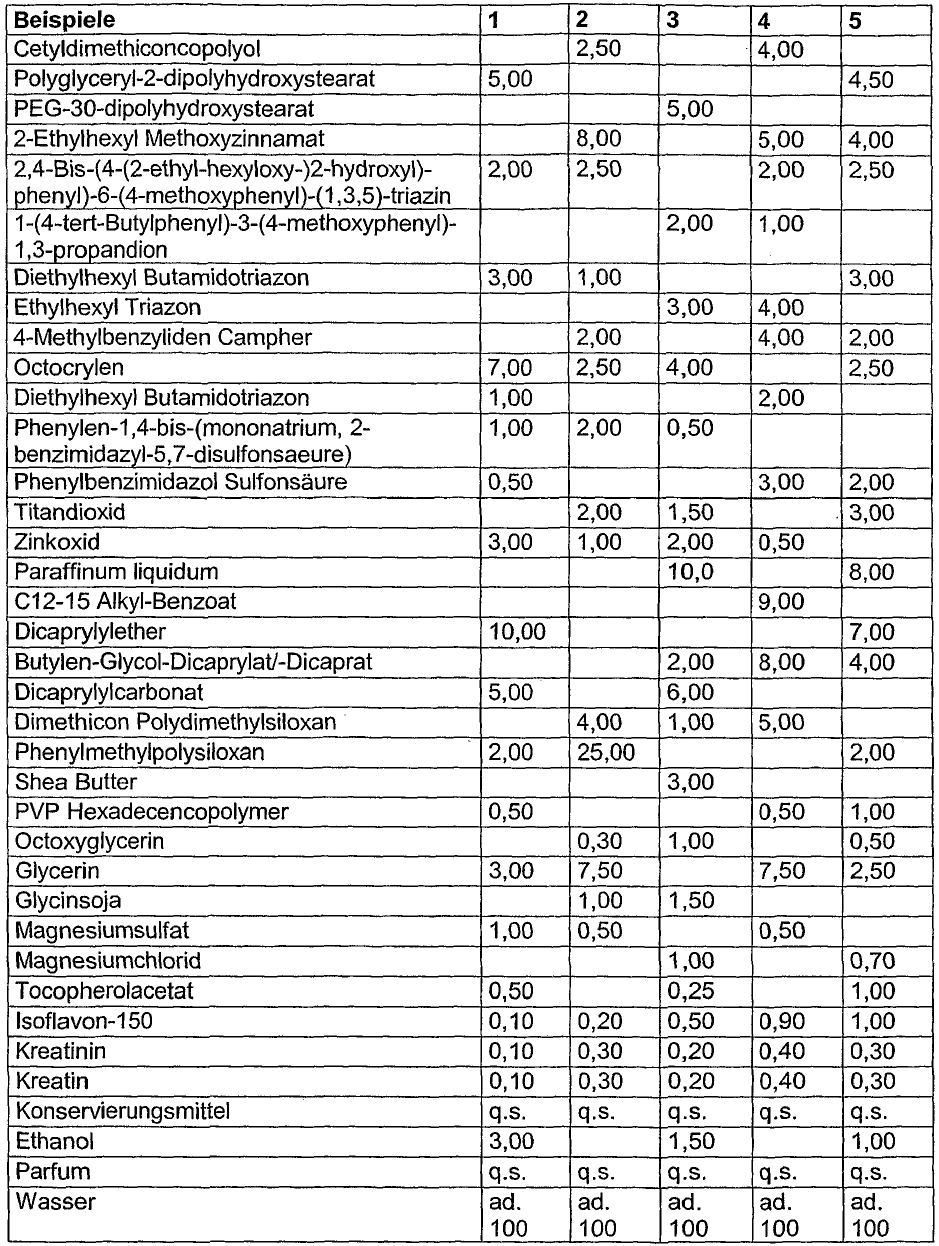

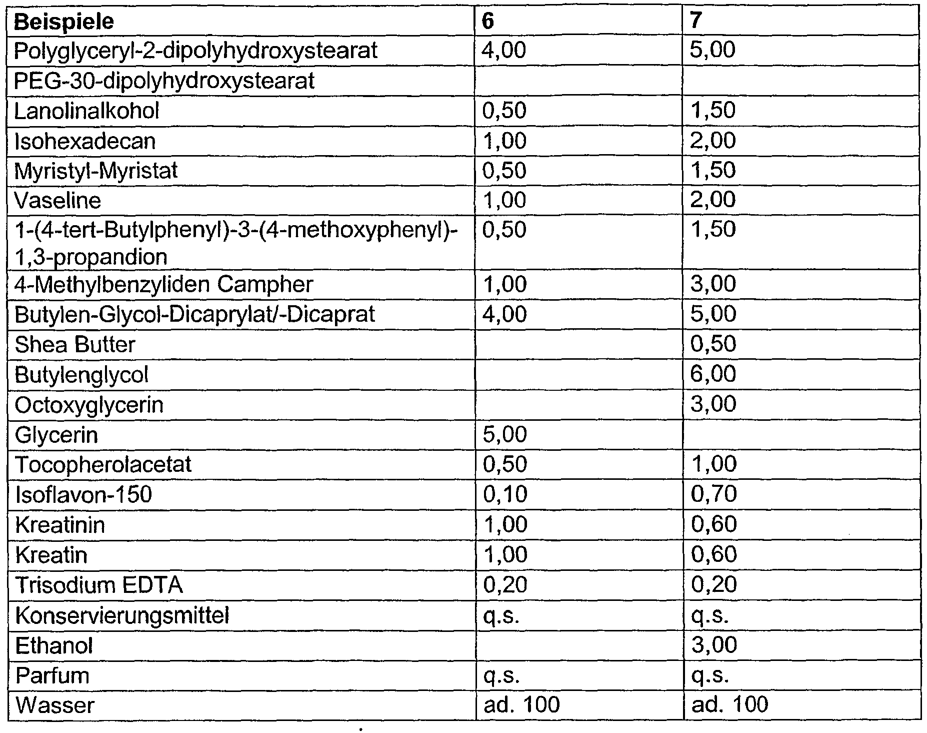

### 4. Hydrodispersionen

| **Beispiele** | **1** | **2** | **3** | **4** | **5** |
|---|---|---|---|---|---|
| Polyoxyethylen(20)cetylstearylether | 1,00 | | | 0,5 | |
| Cetylalkohol | | | 1,00 | | |
| Natriumpolyacrylat | | 0,20 | | 0,30 | |
| Acrylate /C10-30-Alkyl-Acrylat Crosspolymer | 0,50 | | 0,40 | 0,10 | 0,10 |
| Xanthan Gummi | | 0,30 | 0,15 | | 0,50 |
| 2-Ethylhexyl Methoxyzinnamat | | | | 5,00 | 8,00 |
| 2,4-Bis-(4-(2-ethyl-hexyloxy-)2-hydroxyl)-phenyl)-6-(4-methoxyphenyl)-(1,3,5)-triazin | | 1,50 | | 2,00 | 2,50 |
| 1-(4-tert-Butylphenyl)-3-(4-methoxyphenyl)-1,3-propandion | 1,00 | | 2,00 | | |
| Diethylhexyl Butamidotriazon | | 2,00 | 2,00 | 2,00 | 1,00 |
| Ethylhexyl Triazon | 4,00 | | 3,00 | 4,00 | |
| 4-Methylbenzyliden Campher | 4,00 | 4,00 | | | 2,00 |
| Octocrylen | | 4,00 | 4,00 | | 2,50 |
| Phenylen-1,4-bis-(mononatrium, 2-benzimidazyl-5,7-disulfonsaeure | 1,00 | | 0,50 | | 2,00 |
| Phenylbenzimidazol Sulfonsäure | 0,50 | | | 3,00 | |
| Titandioxid | 0,50 | | 2,00 | 3,00 | 1,00 |
| Zinkoxid | 0,50 | 1,00 | 3,00 | | 2,00 |
| C12-15 Alkyl Benzoat | 2,00 | 2,50 | | | |
| Dicaprylylether | | 4,00 | | | |
| Butylenglycol-Dicaprylat/-Dicaprat | 4,00 | | 2,00 | 6,00 | |
| Dicaprylylcarbonat | | 2,00 | 6,00 | | |
| Dimethicon Polydimethylsiloxan | | 0,50 | 1,00 | | |
| Phenylmethylpolysiloxan | 2,00 | | | 0,50 | 2,00 |
| Shea Butter | | 2,00 | | | |
| PVP Hexadecencopolymer | 0,50 | | | 0,50 | 1,00 |
| Octoxyglycerin | | | 1,00 | | 0,50 |
| Glycerin | 3,00 | 7,50 | | 7,50 | 2,50 |
| Glycinsoja | | | 1,50 | | |
| Tocopherolacetat | 0,50 | | 0,25 | | 1,00 |
| Isoflavon-150 | 0,3 | 0,10 | 0,50 | 0,30 | 0,20 |
| Kreatinin | 0,10 | 0,30 | 1,00 | 0,70 | 0,50 |
| Kreatin | 0,10 | 0,30 | 1,00 | 0,70 | 0,50 |
| Konservierungsmittel | q.s. | q.s. | q.s. | q.s. | q.s. |
| Ethanol | 3,00 | 2,00 | 1,50 | | 1,00 |
| Parfum | q.s. | q.s. | q.s. | q.s. | q.s. |
| Wasser | ad. 100 | ad. 100 | ad. 100 | ad. 100 | ad. 100 |

### 5. Beispiel (Gelcreme):

| | |
|---|---|
| Acrylat /C10-30 Alkylacrylat Crosspolymer | 0,40 |
| Polyacrylsaeure | 0,20 |
| Xanthan Gummi | 0,10 |
| Cetearylalkohol | 3,00 |
| C12-15 Alkylbenzoat | 4,00 |
| Caprylic/Capric Triglycerid | 3,00 |
| Cyclisches Dimethylpolysiloxan | 5,00 |
| Dimeticon Polydimethylsiloxan | 1,00 |
| Isoflavon-150 | 0,10 |
| Kreatinin | 0,30 |
| Kreatin | 0,30 |
| Glycerin | 3,00 |
| Natriumhydroxid | q.s. |
| Konservierungsmittel | q.s. |
| Parfum | q.s. |
| Wasser | ad 100,0 |
| pH-Wert eingestellt auf 6.0 | |

### 6. Beispiel (W/O-Creme)

| | |
|---|---|
| Polyglyceryl-3-Diisostearate | 3,50 |
| Glycerin | 3,00 |
| Polyglyceryl-2-Dipolyhydroxystearate | 3,50 |
| Isoflavon-150 | 0,20 |
| Kreatinin | 0,10 |
| Kreatin | 0,10 |
| Konservierungsmittel | q.s. |
| Parfum | q.s. |
| Wasser | ad 100,0 |
| Magnesiumsulfat | 0,6 |
| Isopropylstearat | 2,0 |
| Caprylylether | 8,0 |
| Cetearylisononanoat | 6,0 |

### 7. Beispiel (W/O/W-Creme):

| | |
|---|---|
| Glycerylstearat | 3,00 |
| PEG-100-Stearat | 0,75 |
| Behenylalkohol | 2,00 |
| Caprylic-/Capric-Triglycerid | 8,0 |
| Octyldodecanol | 5,00 |
| C12-15 Alkylbenzoat | 3,00 |
| Isoflavon-150 | 1,00 |
| Kreatinin | 1,00 |
| Kreatin | 1,00 |
| Magnesiumsulfat (MgSO4) | 0,80 |
| Ethylendiamintetraessigsaeure | 0,10 |
| Konservierungsmittel | q.s. |
| Parfum | q.s. |
| Wasser | ad 100,0 |
| pH-Wert eingestellt auf 6.0 | |

## Patentansprüche

1. Kosmetische oder dermatologische Zubereitungen mit einem Gehalt an einer Wirkstoffkombination aus
(a) Kreatinin
(b) Kreatin und
(c) Sojabohnenkeimextrakten.

2. Kosmetische oder dermatologische Zubereitungen nach Anspruch 1 **dadurch gekennzeichnet, daß** der Gehalt an Kreatinin 0,001 - 50 Gew.-%, vorzugsweise 0,1 - 10 Gew.-% sowie der Gehalt an Kreatin 0,001 - 50 Gew. %, vorzugsweise 0,1 - 10 Gew. %, bezogen auf das Gesamtgewicht der Zubereitungen, beträgt.

3. Zubereitungen nach einem der vorangehenden Ansprüche **dadurch gekennzeichnet, daß** die Zubereitungen 0,1 bis 20 Gew. -%, bevorzugt 0,5 - 10 Gew. -%, ganz besonders bevorzugt 1 - 5 Gew. -% Sojabohnenkeimextrakte enthalten.

4. Zubereitungen nach einem der vorangehenden Ansprüche zur Behandlung und Prophylaxe der schädlichen Auswirkung ultravioletter Strahlung auf die Haut.

5. Zubereitungen nach einem der Ansprüche 1 bis 3 zur Erleichterung der Vernarbung bei verringerter Narben bildung bei Verletzungen der Oberhaut.

6. Nicht therapeutische Verwendung von Zubereitungen nach einem der Ansprüche 1 bis 3 zur Behandlung und Prophylaxe von Alterflacken, Falten, Hautschlaffheit und Elastizitäts verlust der Haut.

## Claims

1. Cosmetic or dermatological preparations containing an active ingredient combination of
a) creatinine
b) creatine and
c) soya bean germ extracts.

2. Cosmetic or dermatological preparations according to Claim 1, **characterized in that** the content of creatinine is 0.001-50% by weight, preferably 0.1-10% by weight, and the content of creatine is 0.001-50% by weight, preferably 0.1-10% by weight, based on the total weight of the preparations.

3. Preparations according to one of the preceding claims, **characterized in that** the preparations comprise 0.1 to 20% by weight, preferably 0.5-10% by weight, very particularly preferably 1-5% by weight, of soya bean germ extracts.

4. Preparations according to one of the preceding claims for the treatment and prophylaxis of the harmful effect of ultraviolet radiation on the skin.

5. Preparations according to one of Claims 1 to 3 for facilitating healing with reduced scarring for injuries to the epidermis.

6. Nontherapeutic use of preparations according to one of Claims 1 to 3 for the treatment and prophylaxis of age spots, wrinkles, skin looseness and loss of elasticity of the skin.

## Revendications

1. Compositions cosmétiques ou dermatologiques présentant une teneur en une combinaison de substances actives constituée par
a) de la créatinine
b) de la créatine et
c) des extraits de germes de soja.

2. Compositions cosmétiques ou dermatologiques selon la revendication 1, **caractérisées en ce que** la teneur en créatinine est de 0,001 - 50% en poids, de préférence de 0,1 - 10% en poids et la teneur en créatine est de 0,001 - 50% en poids, de préférence de 0,1 - 10% en poids, par rapport au poids total des compostions.

3. Compositions selon l'une quelconque des revendications précédentes, **caractérisées en ce que** les compositions contiennent 0,1 à 20 % en poids, de préférence 0,5 - 10% en poids, de manière tout particulièrement préférée 1 - 5% en poids d'extraits de germes de soja.

4. Compositions selon l'une quelconque des revendications précédentes destinées au traitement et à la prophylaxie de l'effet nuisible du rayonnement ultraviolet sur la peau.

5. Compositions selon l'une quelconque des revendications 1 à 3 pour faciliter la cicatrisation lors d'une formation diminuée de cicatrices en cas de lésions de l'épiderme.

6. Utilisation non thérapeutique de compositions selon l'une quelconque des revendications 1 à 3 pour le traitement et la prophylaxie de taches de vieillesse, de rides, de la peau relâchée et de la perte d'élasticité de la peau.
